# DEMANDE DE BREVET EUROPEEN

(11) **EP 4 105 934 A1**
(43) Date de publication de la demande: **21.12.2022**
(21) Numéro de dépôt: 21305814.2
(22) Date de dépôt: 14.06.2021
(51) Int. Cl.: G16B 40/20

(54) **PROCÉDÉ DE CLASSIFICATION DE PATIENTS À PARTIR D'ENSEMBLES DE SOUS-SIGNATURES PROPRES À UNE PATHOLOGIE**

(71) Demandeur: Quinten, 75017 Paris (FR)
(72) Inventeur: ZUCKER, Jean-Daniel Olivier, 75014 Paris (FR); QUEYREL, Maxence Paul Vincent, 75011 Paris (FR); TEMPLIER, Alexandre Marie Nicolas, 78700 Conflans Sainte Honorine (FR)
(74) Mandataire: Cabinet Camus Lebkiri

(57) **Abrégé**

Un aspect de l'invention concerne un procédé comprenant les étapes de :
- Réception d'au moins une donnée métagénomique étant le résultat d'une analyse métagénomique d'un échantillon d'un microbiome d'un patient et comprenant au moins une abondance de taxon métagénomique,
- Comparaison de chaque abondance de taxon métagénomique avec chaque sous-signature métagénomique d'au moins un ensemble de sous-signatures métagénomiques propres à une pathologie, chaque sous-signature comprenant au moins une règle relative à une abondance de taxon métagénomique et étant associée à la présence ou à l'absence de la pathologie, l'ensemble de sous-signatures métagénomiques ayant été généré par un procédé de découverte de sous-groupes,
- Si la donnée métagénomique comprend une abondance de taxon métagénomique respectant la règle d'au moins une sous-signature de l'ensemble de sous-signatures métagénomiques, classification du patient dans une classe représentative de la présence ou de l'absence de la pathologie en fonction de l'association de la sous-signature.

## Description

### DOMAINE TECHNIQUE DE L'INVENTION

Le domaine technique de l'invention est celui de la bio-informatique.

La présente invention concerne un procédé de classification de patients à partir de données métagénomiques et en particulier une classification à partir d'un ensemble de sous-signatures métagénomiques ayant été généré par un procédé de découverte de sous-groupes.

### ARRIÈRE-PLAN TECHNOLOGIQUE DE L'INVENTION

Dans le cadre de la médecine de précision, les praticiens ont de plus en plus souvent recours à des diagnostics qui se basent sur des données « omiques » (génomiques, transcriptomiques, radiomiques, etc.), et notamment des données dites métagénomiques. Les données métagénomiques proviennent d'analyses métagénomiques. Une analyse métagénomique permet par exemple l'étude d'un microbiome par la description génomique du contenu d'un prélèvement issu de celui-ci. Cette analyse, lorsqu'elle est réalisée par séquençage direct, comprend un échantillonnage du prélèvement, une extraction de l'Acide Désoxyribo-Nucléique (« ADN ») de l'échantillon, une construction de librairie, et un séquençage afin d'obtenir, après une assignation taxonomique, des tables d'abondances et donc l'identification et la quantification des espèces formant le microbiome étudié. Cette analyse peut par exemple être réalisée en utilisant des plateformes de séquençage haut-débit (« HTS » pour « High-Throughput Sequencing » selon la dénomination anglo-saxonne) telles que celles proposées par Illumina^{®} ou Nanopore^{®}. Il devient ainsi possible d'envisager une médecine de précision intégrant la métagénomique.

On peut par exemple, sur la base de la composition des microbiomes intestinaux de patients diagnostiquer différentes pathologies, comme montré dans [C. Jobin, « Precision medicine using microbiota », Science, 05 Jan 2018: Vol. 359, Issue 6371, pp. 32-34 DOI: 10.1126/science.aar2946], confirmant le rôle clef du microbiote dans les pathologies et l'évaluation des interventions thérapeutiques.

Deux types d'approches principales ont été utilisés pour faire de la prédiction de phénotype à partir de données métagénomiques :
- Des procédés de classification de l'état de l'art tels que les « SVM » pour « Support Vector Machine » en français « Machines à Vecteur de Support », Random Forest, régression logistique pénalisée, plus proches voisins, réseaux de neurones etc ... pour lesquels les données métagénomiques sont prétraitées au préalable pour tirer au mieux partie de la puissance des procédés, voir par exemple [E. Pasolli et al. « Machine Learning Meta-analysis of Large Metagenomic Datasets: Tools and Biological Insights », PLoS Computational Biology 12(7), July 2016 DOI:10.1371/journal.pcbi. 1004977], [G. Zeller et al. "Potential of fecal microbiota for early-stage détection of colorectal cancer", Mol Syst Biol. Nov 2014; 10(11): 766. doi: 10.15252/msb.20145645] ou encore [K. Forslund et al. « Disentangling type 2 diabetes and metformin treatment signatures in the human gut microbiota », Nature, 10 Dec 2015 ;528(7581):262-266, doi: 10.1038/nature15766]. Ces procédés permettent souvent de réduire la dimensionnalité des données avec des méthodes de régularisation et offrent surtout une bonne généralisation sur les données de test en limitant les risques de sur-apprentissage. Cependant, les modèles produits sont généralement complexes et difficiles à interpréter. C'est pourquoi une deuxième famille de procédés tente de résoudre ce problème.
- Des procédés dédiés, adaptés aux données métagénomiques, tels que Predomics [E. Prifti et al. « Interpretable and accurate prediction models for metagenomics data », GigaScience, Volume 9, Issue 3, March 2020, giaa010, https://doi.org/10.1093/gigascience/giaa010] ou GutBalance [F. Yang "GutBalance: a server for the human gut microbiome-based disease prediction and biomarker discovery with compositionality addressed", Briefings in Bioinformatics, bbaa436, https://doi.org/10.1093/bib/bbaa436]. Ces approches s'inspirent des relations entre écosystèmes et des signatures microbiennes. Ils ont pour objectif d'être à la fois précis et plus interprétables que les procédés de pointe plus complexes comme ceux évoqués ci-dessus.

Les performances des procédés de l'état de la technique sur des données « Benchmark », par exemple dans [E. Pasolli et al. « Machine Learning Meta-analysis of Large Metagenomic Datasets: Tools and Biological Insights », PLoS Computational Biology 12(7), July 2016 DOI:10.1371/journal.pcbi. 1004977] et [G. Zeller et al. "Potential of fecal microbiota for early-stage détection of colorectal cancer", Mol Syst Biol. Nov 2014; 10(11): 766. doi: 10.15252/msb.20145645] varient selon les pathologies étudiées, allant d'une précision faible autour de 55% (pour une base de données sur l'obésité) jusqu'à 89% (jeu de données "Cirrhose"). C'est essentiellement la précision et l'« accuracy » qui sont utilisés comme indicateurs de performance de ces techniques. La précision (par rapport à la classe dite « Positif ») est le rapport entre le nombre d'individus de la classe « Positif » correctement prédits, noté TP pour « True Positive », et le nombre total d'individus prédits « Positif », en incluant ceux faussement prédits Positif, notés FP pour « False Positive », dans cette classe, soit TP / (TP + FP). L'« accuracy » est le rapport entre le nombre d'individus correctement prédits pour toutes les classes et le nombre total d'individus, soit (TP + TN) / (TP + TN + FP + FN), avec TN « True Negative » pour « Vrais Négatif » et FN « False Negative » pour « Faux Négatif ».

Les modèles interprétables ont deux propriétés souhaitables : la concision et la lisibilité pour les non-experts. Ils doivent comprendre des opérations simples et être de taille limitée. C'est pourquoi la sortie de ces modèles est représentée par une équation d'un nombre restreint de variables, préférentiellement inférieur à 6, d'abondances bactériennes qui discriminent les individus se trouvant en-dessous ou au-dessus d'un certain seuil. Les modèles de ces techniques, quand ils sont interprétables, correspondent à une explication valable pour tous les individus.

Ainsi, les procédés de l'état de l'art sont tous des "modèles un pour tous", c'est-à-dire que la règle, la formule ou la fonction issue de leur apprentissage est la même pour tous les individus. L'explication de la décision est donc la même pour tous les individus. Mais si la décision peut être précise, au-delà de la classification entre malade et sain, il est admis que la « composition de la communauté microbienne varie davantage chez les individus dysbiotiques que chez les individus sains » (Zaneveld, et al. Stress and stability: applying the Anna Karenina principle to animal microbiomes. Nature Microbiology 2, 1-8 (2017)). Tout porte donc à croire que les phénotypes métagénomiques des malades sont sous-tendus par de multiples sous-signatures différentes qu'il est important d'appréhender pour plus de robustesse, pour une meilleure capacité de généralisation, et pour ouvrir la voie à des thérapies plus ciblées.

Il existe donc un besoin d'avoir un procédé de classification de données métagénomiques qui tout en restant précis et aussi interprétable que les procédés de l'état de l'art, soit adapté aux spécificités de sous-groupes d'individus dont sont issus les données métagénomiques en leur offrant des explications plus personnalisées.

### RESUME DE L'INVENTION

L'invention offre une solution aux problèmes et aux besoins évoqués précédemment, en permettant une classification de données métagénomiques basée sur un ensemble de sous-signatures propres à une pathologies généré par un procédé de découverte de sous-groupes afin d'obtenir un procédé de classification qui soit précis, interprétable et personnalisé.

Un aspect de l'invention concerne un procédé mis en œuvre par ordinateur de classification de patients, le procédé comprenant les étapes de :
- Réception d'au moins une donnée métagénomique, la donnée métagénomique étant le résultat d'une analyse métagénomique d'un échantillon d'un microbiome d'un patient et comprenant au moins une abondance de taxon métagénomique,
- Comparaison de chaque abondance de taxon métagénomique de la donnée métagénomique avec chaque sous-signature métagénomique d'au moins un ensemble de sous-signatures métagénomiques propres à une pathologie, chaque sous-signature comprenant au moins une règle relative à une abondance de taxon métagénomique et étant associée à la présence ou à l'absence de la pathologie, l'ensemble de sous-signatures métagénomiques ayant été généré par un procédé de découverte de sous-groupes,
- Si la donnée métagénomique comprend une abondance de taxon métagénomique respectant la règle d'au moins une sous-signature de l'ensemble de sous-signatures métagénomiques, classification du patient duquel la donnée métagénomique est issue dans une classe représentative de la présence de la pathologie ou dans une classe représentative de l'absence de la pathologie en fonction de l'association de la sous-signature à la présence ou à l'absence de la pathologie.

L'invention permet, au contraire de l'état de l'art, d'appréhender la signature métagénomique de la pathologie non à travers une unique signature ou d'un unique modèle mais comme un ensemble de sous-signatures interprétables.

En utilisant un algorithme de découverte de sous-groupes, l'invention permet d'identifier différentes sous-signatures propres à la pathologie étudiée, chacune classant des sous-groupes d'individus et chacune utilisant un sous-ensemble différent d'abondance de taxons métagénomiques. Ceci permettant de rendre compte du caractère multiple connu des causes de dysbioses métagénomiques et d'offrir une explication personnalisée et par conséquent des perspectives de traitements ciblés tout en proposant de bonnes performances de classification. Une dysbiose est un déséquilibre microbien ou une mauvaise adaptation de l'organisme, associé à un problème médical, par rapport à un individu en bonne santé.

L'invention se distingue notamment de l'état de l'art en ce que la classification est interprétable et personnalisée. En effet la signature, c'est-à-dire la description de la signature métagénomique, n'est pas représentée sous la forme d'une unique signature, d'une formule de régression, ou d'un arbre de décision, au contraire de l'état de l'art. Dans l'invention, la signature est remplacée par un ensemble de sous-signatures, assimilables à des règles, pour différents sous-groupes d'individus. Cette approche permet une explication de la décision de classification non seulement interprétable mais aussi plus "personnalisée" car différente pour différents groupes d'individus, un patient pouvant correspondre à une ou plusieurs des sous-signatures de l'ensemble des sous-signatures.

En outre, grâce à la présence d'au moins un ensemble de sous-signatures, une mesure statistique de crédibilité différente pour chaque sous-signature de l'ensemble de sous-signatures peut être fournie indiquant ainsi un niveau de confiance de son applicabilité, par exemple un rapport des chances (« Odd ratio » selon la dénomination anglo-saxonne) ou score-F1 (« F1-score » selon la dénomination anglo-saxonne) et valeur-p (« p-value » selon la dénomination anglo-saxonne), améliorant ainsi l'interprétabilité du procédé selon l'invention.

Outre les caractéristiques qui viennent d'être évoquées dans le paragraphe précédent, le procédé de classification de patients selon un aspect de l'invention peut présenter une ou plusieurs caractéristiques complémentaires parmi les suivantes, considérées individuellement ou selon toutes les combinaisons techniquement possibles :
- si l'abondance de taxon métagénomique de la donnée métagénomique ne respecte aucune règle d'aucune sous-signature de l'ensemble de sous-signatures métagénomiques, ou, si l'abondance de taxon métagénomique de la donnée métagénomique respecte au moins une première et une deuxième règles respectivement d'une première et une deuxième sous-signatures de l'ensemble de sous-signatures métagénomiques, la première sous-signature étant associée à la présence de la pathologie et la deuxième sous-signature étant associée à l'absence de la pathologie, le patient duquel la donnée métagénomique est issue est classifié par un procédé de classification supervisé.
- l'échantillon provient du microbiote intestinal du patient.
- la pathologie est une cirrhose, un cancer du côlon, un diabète, une maladie de Crohn.
- le procédé comprend, après l'étape de réception de la donnée métagénomique, une étape de pré-traitement de la donnée métagénomique comprenant une transformation log-ratio centrée de la donnée métagénomique.
- le procédé comprend préalablement les étapes suivantes:
   - Une étape de réception d'au moins une donnée métagénomique d'entraînement de contrôle et d'au moins une donnée métagénomique d'entraînement de cas, la au moins une donnée métagénomique d'entraînement de contrôle étant le résultat d'une analyse métagénomique d'un échantillon d'un microbiote d'un patient cas-contrôle et la au moins une donnée métagénomique d'entraînement de cas étant le résultat d'une analyse métagénomique d'un échantillon d'un microbiote d'un patient cas-témoin,
   - Une étape de préparation des données d'entraînement reçues,
   - Une étape de génération de l'ensemble de sous-signatures propres à la pathologie par le premier procédé de découverte de sous-groupes supervisé à partir des données métagénomiques d'entraînement préparées.
- le procédé comprend en outre au moins :
   - Une étape d'optimisation de l'ensemble de sous-signatures propres à la pathologie à partir des données métagénomiques d'entraînement de contrôle pour obtenir au moins un jeu de sous-signatures propres à la pathologie optimisé pour les patients cas-contrôle,
   - Une étape d'optimisation de l'ensemble de sous-signatures propres à la pathologie à partir des données métagénomiques d'entraînement de cas pour obtenir au moins un jeu de sous-signatures propres à la pathologie optimisé pour les patients cas-témoin,
   - Une étape d'assemblage du jeu de sous-signatures propres à la pathologie optimisée pour les patients cas-contrôle et du jeu de sous-signatures propres à la pathologie optimisée pour les patients cas-témoin pour obtenir l'ensemble de sous-signatures propres à la pathologie.
- chaque étape d'optimisation comprend une pluralité de sous-étapes de calcul de métrique, chaque sous-étape de calcul de métrique comprenant :
   - le calcul d'une métrique pour chaque sous-signature propre à la pathologie de l'ensemble de sous-signatures propres à la pathologie et
   - la comparaison du résultat du calcul de ladite métrique avec une règle basée sur un seuil prédéfini,
- chaque sous-étape étant associée à un niveau de contrainte, les sous-étapes se succédant du plus bas niveau de contrainte au plus haut niveau de contrainte, le jeu de sous-signatures propres à la pathologie issu de l'étape d'optimisation comprenant au moins les sous-signatures propres à la pathologie respectant la règle basée sur un seuil prédéfini pour chaque sous-étape de la pluralité de sous-étapes.
- chaque étape d'optimisation comprend en outre:
   - Une sous-étape de sélection d'une première sous-signature parmi les sous-signatures comprises dans l'ensemble de sous-signatures propres à la pathologie, la première sous-signature sélectionnée étant la sous-signature maximisant le résultat du calcul d'une métrique prédéfinie parmi les métriques calculées aux sous-étapes de calcul de métriques des étapes d'optimisation,
   - Pour chaque autre sous-signature de l'ensemble de sous-signatures propres à la pathologie différente de la première sous-signature sélectionnée :
      - Une sous-étape de calcul de la métrique prédéfinie pour un groupe de sous-signatures comprenant la première sous-signature et l'autre sous-signature,
      - Si la différence entre le résultat du calcul de la métrique pour le groupe de sous-signatures et le résultat du calcul de la métrique pour la première sous-signature respecte une règle basée sur un seuil de différence prédéfini, l'autre sous-signature est ajoutée au jeu de sous-signatures propres à la pathologie optimisé.

Un autre aspect de l'invention concerne un produit-programme d'ordinateur comprenant des instructions qui, lorsque le programme est exécuté par un ordinateur, conduisent celui-ci à mettre en œuvre les étapes du procédé de classification de patients selon l'invention.

Encore un autre aspect de l'invention concerne un support d'enregistrement lisible par ordinateur comprenant des instructions qui, lorsqu'elles sont exécutées par un ordinateur, conduisent celui-ci à mettre en œuvre les étapes du procédé de classification de patients selon l'invention.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La figure 1 montre une représentation schématique du procédé de classification de patients à partir de données métagénomiques selon l'invention,
- La Figure 2 montre une représentation schématique d'une étape de génération d'ensemble de sous-signatures du procédé de classification selon l'invention,
- La Figure 3 montre une représentation schématique de sous-étapes d'optimisation du procédé de classification selon l'invention,
- La Figure 4 montre une représentation schématique des différences entre le procédé selon l'invention et l'état de l'art.

### DESCRIPTION DÉTAILLÉE

Les figures sont présentées à titre indicatif et nullement limitatif de l'invention.

Sauf précision contraire, un même élément apparaissant sur des figures différentes présente une référence unique.

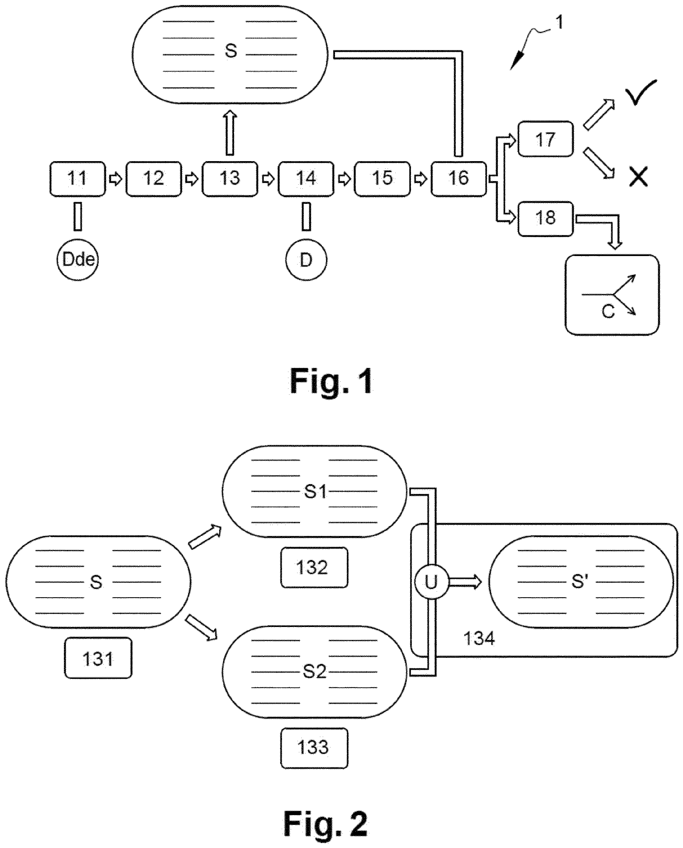

La Figure 1 montre une représentation schématique d'un procédé de classification de patients selon l'invention.

Le procédé 1 de classification de patients selon l'invention comprend huit étapes 11 à 18.

Les étapes 11 à 13 sont des étapes d'entraînement du procédé de classification 1, notamment afin d'obtenir au moins un ensemble de sous-signatures métagénomiques propres à une pathologie à partir de données métagénomiques d'entraînement « Dde ».

Le procédé de classification 1 selon l'invention est mis en œuvre par un ordinateur comprenant un processeur et une mémoire, la mémoire comprenant des instructions qui, lorsqu'elles sont exécutées par le processeur, conduisent l'ordinateur à mettre en œuvre les étapes du procédé 1 selon l'invention.

Une première étape 11 est une étape de réception d'au moins une donnée métagénomique d'entraînement de contrôle et d'au moins une donnée métagénomique d'entraînement de cas. La réception des données métagénomiques d'entraînement peut par exemple être réalisée via une interface réseau de l'ordinateur mettant en œuvre le procédé 1, ou via une connexion directe entre la mémoire stockant la donnée et le processeur de l'ordinateur. La réception est comprise dans une transmission qui peut être réalisée au sein d'un réseau, par exemple via une connexion filaire suivant par exemple les protocoles USB^{®} ou Ethernet^{®}, ou par exemple via une connexion sans-fil suivant par exemple les protocoles Wi-Fi^{®}, Bluetooth^{®}, LoRa^{®} ou tout autre protocole de transfert de données sans fil.

On entend par « donnée métagénomique » une donnée issue d'une analyse métagénomique d'un échantillon d'un microbiote d'un patient. Une telle donnée permet d'identifier les différentes espèces formant le microbiote duquel l'échantillon est issu et est par exemple une table d'abondance. Un échantillon issu du microbiote d'un patient provient d'un prélèvement. L'invention est applicable à tout microbiote humain, par exemple l'invention est applicable au microbiote intestinal, buccal, vaginal, pénien, placentaire ou cutané.

L'étape 11 comprend ainsi la réception d'au moins deux données : au moins une donnée métagénomique d'entraînement de contrôle, correspondant à une donnée métagénomique d'entraînement issue du microbiote d'un patient sain, aussi appelé cas-contrôle et au moins une donnée métagénomique d'entraînement de cas, correspondant à une donnée métagénomique d'entraînement issue du microbiote d'un patient atteint d'une pathologie étudiée, aussi appelé cas-témoin. Les pathologies étudiées peuvent être, de façon non limitative, la cirrhose, le diabète, le cancer du côlon, la maladie de Cröhn, ou toute autre pathologie étudiable à l'aide du procédé selon l'invention. Le procédé de classification 1 comprend donc un entraînement supervisé, en ce que les données métagénomiques d'entraînement sont « étiquetées » comme étant issues du microbiote d'un patient atteint de la pathologie étudiée ou d'un patient non atteint de la pathologie étudiée. Cet « étiquetage » peut être réalisé en adjoignant aux données un label indiquant le type de patient « contrôle » ou « cas ». Préférentiellement, les données métagénomiques d'entraînement « Dde » proviennent d'une cohorte d'au moins une centaine de patients comprenant des patients « cas-contrôle » et « cas-témoin » répartis équitablement ou en partie.

Le procédé de classification 1 comprend une étape 12 de préparation de données métagénomiques d'entraînement. Cette préparation peut comprendre une réduction de dimensionnalité et/ou une normalisation. Par exemple, l'étape 12 de préparation de données métagénomiques d'entraînement peut comprendre une transformation log-ratio centrée (« centered-log » ratio - clr - selon la dénomination anglo-saxonne) des données métagénomiques d'entraînement, les données métagénomiques étant des données compositionnelles. La transformation log-ratio centrée a pour avantage d'enlever la restriction de la plage de valeurs des données compositionnelles et de conserver la même dimensionnalité / les mêmes variables initiales, ce qui est indispensable pour avoir un modèle interprétable.

Le procédé de classification 1 comprend une étape 13 de génération d'au moins un ensemble S de sous-signatures propres à la pathologie étudiée par un procédé de découverte de sous-groupes à partir des données métagénomiques d'entraînement de dimension réduite obtenues à l'étape 12.

Le procédé de découverte de sous-groupes peut être tout procédé de découverte de sous-groupes connu, préférentiellement le procédé de découverte de sous-groupes QFinder^{®} décrit dans [C. Esnault et al, « Q-Finder: An Algorithm for Credible Subgroup Discovery in Clinical Data Analysis - An Application to the International Diabetes Management Practice Study », Front. Artif. Intell., 17 December 2020, doi:10.3389/frai.2020.559927] à la partie 2.2 « Preprocessing and Candidate Subgroups Generation in Q-Finder ». On entend par « procédé de découverte de sous-groupes » un procédé prenant en entrée une pluralité de données et permettant de proposer en sortie une identification de groupes de données pertinents au sein de ces données. Un tel procédé est aussi appelé procédé d'identification de sous-groupes ou « subgroup discovery » selon la dénomination anglo-saxonne, ou encore « procédé de découverte de modèles exceptionnels locaux ». Un sous-groupe identifié par un procédé de découverte de sous-groupes est caractérisé par son extension, c'est-à-dire l'ensemble d'objets qu'il comprend, par exemple des identifiants de patients, et par son intention, c'est-à-dire une description qui caractérise les objets du sous-groupe, par exemple « Toute les femmes adultes ».

Le résultat du procédé de découverte de sous-groupes est un ensemble S de sous-signatures propres à une pathologie, car les données d'entraînement « Dde » en entrée du procédé de découverte de sous-groupe sont des données soit liées à un patient sain (« contrôle »), soit liées à un patient atteint de la pathologie (« cas »), les données d'entraînement « Dde » étant annotées. On entend par « sous-signature » un sous-groupe identifié par le procédé de découverte de sous-groupes. Une sous-signature est liée à une signature métagénomique de la pathologie. Des différences significatives de proportion ou dans les abondances relatives de plusieurs taxons (les microbes appartenant à une catégorie taxonomique, généralement de faible niveau comme le genre ou l'espèce) bactériens qui sont en corrélation avec la présence d'une pathologie définissent une signature métagénomique spécifique de cette pathologie. Une signature permet, à partir de la quantification des abondances relatives des gènes ou espèces, de construire un prédicteur de la pathologie considérée. On parle aussi de biomarqueur de la pathologie. Une sous-signature est donc une signature caractérisant un sous-groupe d'individus ayant ou non la pathologie. Une sous-signature comprend au moins une règle relative à une abondance de taxon métagénomique dans la donnée métagénomique et est associée à la présence ou à l'absence de pathologie chez le patient. Des exemples de sous-signatures résultant d'un entraînement d'un procédé de découverte de sous-groupes avec des données d'entraînement métagénomiques Dde issues du microbiote intestinal de 232 patients d'entraînement dont 118 patients cas atteints de cirrhose et 114 patients contrôles sont :
- RP1 : Si Veillonella_unclassified >= 7.27e-04, alors Cirrhotique avec F1-score = 0.94 et p-value hypergéométrique = 2.05e-09
- RP2 : Si Streptococcus_anginosus >= 1.85e-04, alors Cirrhotique avec F1-score = 0.70 et p-value hypergéométrique = 2.05e-05
- RP3 : Si Veillonella_dispar >= 3.64e-04, alors Cirrhotique avec F1-score = 0.86 et p-value hypergéométrique = 7.37e-08
- RP4 : Si Veillonella_parvula >= 1.22e-03, alors Cirrhotique avec F1-score = 0.75 et p-value hypergéométrique = 2.05e-05.
- RP5 : Si Megasphaera_micronuciformis >= 2.90e-06, alors Cirrhotique avec F1-score = 0.77 et p-value hypergéométrique = 2.90e-06
- RN1 : Si Veillonella_unclassified <= 2.11e-03, alors Sain avec F1-score = 0.92 et p-value hypergéométrique = 6.31e-09
- RN2 : Si Veillonella_dispar <= 6.28e-05, alors Sain avec F1-score = 0.79 et p-value hypergéométrique = 2.19e-05

Avec « RP » pour « Règle Positive » les sous-signatures correspondant à une présence de pathologie et « RN » pour « Règle Négative » les sous-signatures correspondant à une absence de pathologie. L'ensemble comprenant les règles RP1 à RP5, RN1 et RN2 est l'ensemble S de sous-signatures métagénomiques propres à la pathologie « Cirrhose », chaque sous-signature comprenant au moins une règle relative à une abondance de taxon métagénomique dans la donnée métagénomique, étant associée à la présence ou à l'absence de la pathologie et comprenant une mesure statistique de crédibilité, grâce à l'utilisation du procédé de découverte de sous-groupes QFinder^{®}. Par exemple, la règle RP1 se lit comme suit : si une abondance du taxon métagénomique Veillonella unclassified supérieure à 7.27e-04 est retrouvée dans la donnée métagénomique du patient, alors le patient est cirrhotique. Pour cette sous-signature, les métriques associées sont un score-F1 de 0.94 et une valeur-p hypergéométrique de 2.05e-09.

L'étape 13 comprend quatre sous-étapes 131 à 134. L'étape 13 est représentée à la Figure 2.

La première sous-étape 131 comprend la génération d'un ensemble S de sous-signatures propres à la pathologie étudiée par un procédé de découverte de sous-groupes à partir de données métagénomiques d'entraînement Dde comme décrit précédemment, par exemple par la partie « Preprocessing and Candidate Subgroups Génération » de QFinder^{®}.

La seconde sous-étape 132 comprend l'optimisation des sous-signatures de l'ensemble S de sous-signatures pour les données d'entraînement issues de patients « cas ». Cette optimisation comprend plusieurs sous-étapes de calcul de métriques et permet d'obtenir un premier ensemble optimisé S1 de sous-signatures propres à la pathologie étudiée pour les patients atteints par la pathologie.

La troisième sous-étape 133 comprend l'optimisation des sous-signatures de l'ensemble S de sous-signatures pour les données d'entraînement issues de patients « contrôle ». Cette optimisation comprend plusieurs sous-étapes de calcul de métriques et permet d'obtenir un second ensemble optimisé S2 de sous-signatures propres à la pathologie étudiée pour les patients sains.

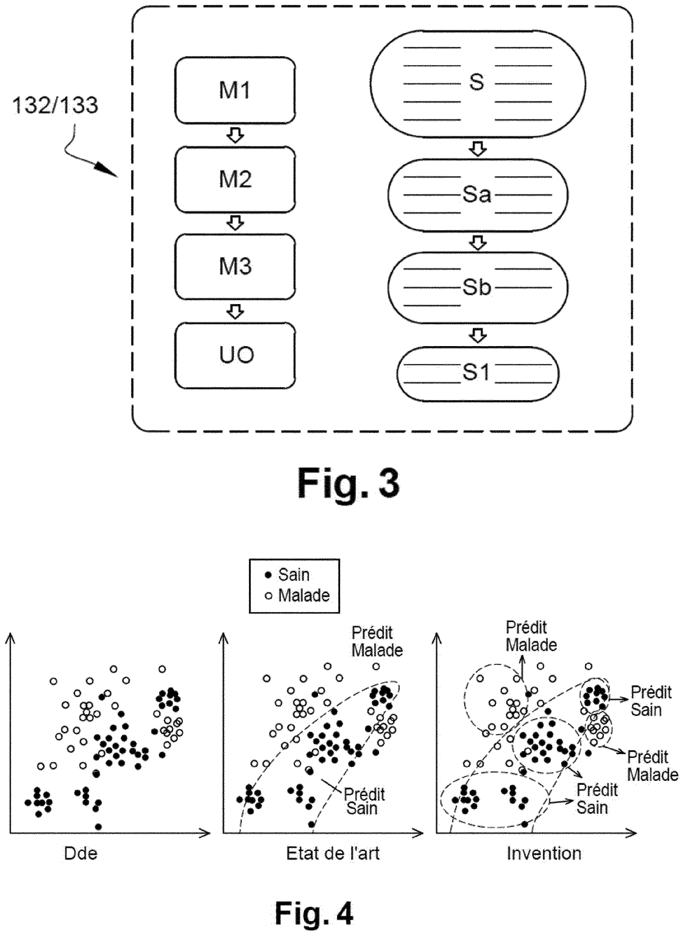

Un exemple de réalisation de la sous-étape 132 est représenté à la Figure 3. La Figure 3 montre une représentation schématique d'une optimisation d'un ensemble de sous-signatures S afin d'obtenir un jeu de sous-signatures optimisé S1. La Figure 3 s'applique de la même manière à une optimisation selon la troisième sous-étape 133 pour obtenir un jeu de sous-signatures optimisé S2.

Une sous-étape d'optimisation selon l'invention comprend une pluralité de sous-étapes de calcul de métriques et de comparaison du résultat du calcul à une règle basée sur un seuil prédéfini. Par exemple, la Figure 3 montre quatre sous-étapes M1 à M4. Les métriques sont calculées pour les sous-signatures appliquées aux données d'entraînement « Dde » de cas à la sous-étape 132 ou de contrôle à la sous-étape 133. Cela permet d'avoir des jeux de sous-signatures optimisés pour chacun des types de patients amenés à être classés.

Chaque sous-étape M1 à M4 comprend le calcul d'une métrique, par exemple parmi les métriques suivantes, ordonnées selon leur niveau de contrainte, du plus bas au plus haut niveau de contrainte :
- 1. Couverture : taille de la sous-signature / taille de l'ensemble de sous-signatures,
- 2. Score-F1 : (2 * TP) / (2 * TP + FN + FP), avec TP le nombre de vrais positifs (« True Positive »), FN le nombre de faux négatifs (« False Negative ») et FP le nombre de faux positifs (« False Positive »).
- 3. Influence sur le score-F1 : calcule la différence et le ratio entre le score-F1 de la sous-signature et le score-F1 de la sous-signature auquel un critère a été omis. Cela permet de s'assurer que le critère en question joue un rôle suffisamment important sur le score du sous-groupe. On entend par « critère» un ensemble comprenant au moins une variable et au moins un valeur de filtre pour cette variable. Par exemple, un critère peut être sexe=homme ou age>19.
- 4. Valeur-P hypergéométrique : est calculée comme la somme des fonctions de probabilité de masse de la loi hypergéométrique dans l'intervalle [TP, TP+FP] (voir par exemple scipy.stats.hypergeom de l'écosystème Python SciPy^{®}).
- 5. Correction de Bonferroni de la valeur-p hypergéométrique : La valeur-p est multipliée par le nombre de sous-signatures créées.

Chaque sous-étape de calcul de métrique est associée à un niveau de contrainte, les sous-étapes se succédant du plus bas niveau de contrainte au plus haut niveau de contrainte. A chaque sous-étape de calcul de métrique, le résultat du calcul de ladite métrique pour chaque sous-signature de l'ensemble de sous-signatures est comparé avec une règle basée sur un seuil prédéfini, comme par exemple une couverture supérieure à 10% et une valeur-p inférieure à 5%. Lorsque le résultat du calcul d'une métrique pour une sous-signature ne respecte pas une règle, la sous-signature est retirée de l'ensemble de sous-signatures, créant ainsi un jeu de sous-signatures comprenant un nombre inférieur de sous-signatures au nombre de sous-signatures de l'ensemble de sous-signatures S. Par exemple, à chaque sous-étape M1 à M3 de la Figure 3, l'ensemble S de sous-signatures se voit retirer des sous-signatures, créant à chaque fois un nouveau jeu de sous-signatures Sa puis Sb, obtenant ainsi un jeu de sous-signatures Sb optimisé. Ainsi, les sous-signatures comprises dans le jeu de sous-signatures Sb optimisé ne comprend que des sous-signatures ayant respecté toutes les règles évaluées aux sous-étapes M1 à M3, chaque règle étant basée sur un seuil lié au résultat du calcul de la métrique à la sous-étape correspondante. Ainsi, en calculant la couverture à la sous-étape M1, seules les sous-signatures ayant une couverture supérieure à un seuil prédéfini seront évaluées par rapport à la métrique calculée à la sous-étape suivante, c'est-à-dire une métrique de niveau de contrainte supérieur.

Les étapes 132 et 133 comprennent en outre, après les sous-étapes M1 à M3 de calcul de métrique, une sous-étape UO d'union optimale des sous-signatures du jeu de sous-signatures Sb optimisé, pour obtenir un jeu de sous-signatures optimisé S1. On entend par « union optimale » une union de sous-signatures maximisant une métrique prédéfinie, par exemple le score-F1 ou la précision. Pour cela, la sous-étape UO prend en entrée les sous-signatures optimisées du jeu optimisé Sb ainsi que le résultat du calcul de la métrique pour chaque sous-signature pour la métrique prédéfinie, obtenue par exemple aux sous-étapes précédentes M1 à M3. La métrique prédéfinie est choisie en fonction du score à maximiser. Par exemple, pour optimiser une erreur de type 1, la précision sera la métrique à maximiser choisie, et pour une erreur de type 2 le rappel sera la métrique à maximiser choisie. Enfin, pour un compromis entre les deux, le score-F1 sera choisi.

Cette sous-étape d'union optimale UO comprend une première étape d'initialisation puis une boucle comprenant deux étapes appelées « avant » et « arrière ». Dans l'étape d'initialisation, une première sous-signature correspondant à la sous-signature maximisant la métrique prédéfinie est sélectionnée. Cette sous-signature appartiendra alors à l'ensemble S1 de sous-signatures résultant de l'union optimale. Ensuite, parmi les sous-signatures restantes appartenant au jeu de sous-signatures optimisé Sb, la sous-signature maximisant la métrique pour son union avec la première sous-signature est sélectionnée. C'est l'étape « avant ». Puis, lorsqu'une autre sous-signature a été sélectionnée à l'étape « avant », l'étape « arrière » comprend le calcul de la métrique pour l'ensemble de sous-signatures résultant de l'union en omettant une des sous-signatures, cela étant réitéré pour chaque sous-signature de l'union. Si le résultat du calcul de la métrique est meilleur sans une sous-signature, cette sous-signature est supprimée de l'union et n'appartiendra donc pas à l'ensemble S1 de sous-signatures résultant de la sous-étape UO d'union optimale. Par exemple, lorsqu'une deuxième sous-signature est sélectionnée pour être ajoutée à la première sous-signature dans l'union optimale des sous-signatures du jeu de sous-signatures optimisé Sb, si le résultat du calcul de la métrique de l'union des deux sous-signatures est meilleur que le résultat du calcul de la métrique pour la première sous-signature seule, la seconde sous-signature est gardée dans l'union. Sinon, elle est supprimée de l'union S1. Les étapes « avant » et « arrière » sont réitérées jusqu'à ce qu'aucune sous-signature n'améliore la métrique de l'union optimale par son ajout ou son retrait à l'union optimale. On entend par « l'ajout de la sous-signature améliore la métrique » ou « le résultat du calcul est meilleur » que la différence entre le résultat du calcul de la métrique avec la sous-signature et le résultat du calcul de la métrique sans la sous-signature respecte une règle basée sur un seuil de différence prédéfini. Par exemple, on peut considérer que la couverture de l'union de sous-signatures est meilleure avec une certaine sous-signature que sans celle-ci si la couverture est supérieure avec la sous-signature que sans. La règle serait alors respectée lorsque la différence de couverture entre l'union avec sous-signature et l'union sans-signature est supérieure à un seuil prédéfini de 0. On obtient ainsi un jeu de sous-signatures optimisé S1 à l'étape 132 et S2 à l'étape 133.

Enfin, la quatrième sous-étape 134 comprend l'union U des deux ensembles de sous-signatures optimisés S1 et S2 respectivement pour les patients atteints par la pathologie et pour les patients sains. Cela permet d'obtenir un ensemble optimisé S' de sous-signatures propres à la pathologie étudiée pour tout patient.

Cette quatrième sous-étape 134 a pour objectif de créer un ensemble de sous-signatures optimisé S' étant l'union optimale des jeux de sous-signatures optimisés S1 et S2 obtenus aux sous-étapes 132 et 133.

Les trois sous-étapes d'optimisation 132 à 134 sont optionnelles. Lorsqu'elles sont mises en œuvre, l'ensemble de sous-signatures utilisé pour la classification est l'ensemble optimisé S' de sous-signatures. Lorsqu'elles ne sont pas mises en œuvre, l'ensemble de sous-signatures utilisé pour la classification est l'ensemble S de sous-signatures généré à la sous-étape 131. Par la suite, pour décrire les étapes de classification 14 à 18, on fera référence à l'un ou l'autre de ces ensembles S ou S' par « ensemble S de sous-signatures ».

Les étapes 14 à 18 sont les étapes de classification de patient duquel la donnée métagénomique est issue, la classification étant basée sur l'ensemble S de sous-signatures obtenu par apprentissage lors des étapes 11 à 13. Les étapes d'apprentissage 11 à 13 peuvent être réalisées à distance, c'est-à-dire par un autre ordinateur ou système que l'ordinateur ou système mettant en œuvre les étapes 14 à 18 de classification. Dans un tel cas, l'ensemble S de sous-signatures est reçu par l'ordinateur, le dispositif ou le système mettant en œuvre les étapes 14 à 18 de classification lors d'une étape non représentée de réception de l'ensemble S de sous-signatures. Ainsi, les étapes 11 à 13 et les étapes 14 à 18 ne sont pas nécessairement mises en œuvre par le même ordinateur.

Le procédé de classification 1 comprend une étape 14 de réception d'au moins une donnée métagénomique D à classifier. Cette étape 14 de réception peut être réalisée de la même manière que l'étape de réception 11 de données métagénomiques d'entraînement Dde. La donnée métagénomique D est de même format que les données d'entraînement Dde, afin de pouvoir la classifier en utilisant les sous-signatures issues de l'apprentissage sur le même format de données. La classification d'une donnée métagénomique permet de classer le patient dont cette donnée est issue, par exemple dans une classe correspondant à la présence d'une pathologie ou dans une classe correspondant à l'absence d'une pathologie. L'invention, en utilisant un procédé de découverte de sous-groupe et donc un ensemble de sous-signatures, permet une interprétabilité de cette classification, c'est-à-dire qu'il permet d'expliquer cette classification ainsi que la crédibilité de cette explication au contraire des procédés de l'état de l'art.

Dans une seconde étape 15, une préparation des données métagénomiques est réalisée. Par exemple, une réduction de dimensionnalité, et / ou une normalisation sont appliquées à la donnée métagénomique reçue. Les mêmes opérations de préparation, par exemple une réduction de dimensionnalité et / ou une normalisation sont utilisées à l'étape 15 que lors de l'apprentissage à l'étape 12.

Dans une troisième étape 16, on compare chaque abondance de taxon métagénomique de la donnée métagénomique avec chaque sous-signature de l'ensemble S de sous-signatures métagénomiques propres à une pathologie. On entend par « comparer une abondance avec une sous-signature » la vérification que l'abondance de taxon métagénomique respecte la ou les règle(s) comprise(s) dans la sous-signature. Par exemple, une abondance de taxon métagénomiques d'une donnée métagénomique D provenant du microbiote intestinal d'un patient respecte la règle RP3 présentée précédemment si le taxon métagénomique Veillonella dispar est présent en abondance supérieure à 3.64e-04 dans la donnée métagénomique D.

Si la donnée métagénomique comprend au moins une abondance de taxon métagénomique respectant la ou les règle(s) d'au moins une sous-signature de l'ensemble de sous-signatures métagénomiques, l'étape 17 de classification de la donnée métagénomique D et donc du patient dont la donnée est issue est réalisée. La classification de la donnée métagénomique D est réalisée en fonction de l'association à la présence ou à l'absence de la pathologie de la sous-signature dont la règle est respectée par l'abondance de taxons dans la donnée métagénomique D, c'est-à-dire que la donnée métagénomique est classifiée dans une classe correspondant à la présence de la pathologie si la sous-signature (règle) est associée à la présence de la pathologie, ou la donnée métagénomique est classifiée dans une classe correspondant à l'absence de la pathologie si la sous-signature (règle) est associée à l'absence de la pathologie. Cela est représentée à la Figure 4, qui montre la différence entre la classification réalisée selon une approche classique de l'état de l'art par rapport à celle réalisée selon l'invention. La Figure 4 montre ainsi une représentation schématique du résultat de la classification dans le cas d'un procédé de l'état de l'art et du procédé selon la présente invention.

A la Figure 4, le procédé de classification de l'état de l'art représenté est un procédé d'apprentissage automatique supervisé tel que des Machines à Vecteur de Support ou XGBoost etc. Comme montré à la Figure 4, les données d'entraînement « Dde » dans l'état de l'art sont annotées, puis le modèle est entraîné avec ces données afin d'obtenir une séparation entre les patients présentant la pathologie et ceux ne la présentant pas. La classification de données se contente ensuite de dire si le patient classé présente la pathologie ou non, sans expliquer cette classification.

Au contraire, dans la présente invention, le modèle après entraînement est un ensemble de sous-groupes, chaque sous-groupe étant divisé en patients présentant la pathologie « Prédit malade » et en patients ne présentant pas la pathologie « Prédit sain ». Ainsi, lors de la classification d'un patient, ce dernier sera classifié dans un sous-groupe et comme présentant ou non la pathologie au sein de ce sous-groupe. Cela permet une interprétabilité des résultats, chaque sous-signature générée par le procédé de découverte de sous-groupe et optimisée aux sous-étapes 132 à 134 comprenant des métriques de crédibilité permettant ainsi d'évaluer la crédibilité de la classification, la classification étant basée sur au moins une règle accessible à un utilisateur du procédé.

On entend par classification dans une « classe correspondant à la présence d'une pathologie » et par classification dans une « classe correspondant à l'absence d'une pathologie » la prédiction d'un label ou d'une étiquette associée à la donnée métagénomique, le label ou l'étiquette correspondant à la classe dans laquelle la donnée a été classée. Par exemple, ce label ou cette étiquette peuvent être « oui » ou « non » ou « présence » ou « absence ». Le label ou l'étiquette peut aussi prendre la forme d'un booléen, d'un chiffre ou toute autre forme permettant de représenter ou de faire comprendre la présence ou l'absence d'une pathologie.

Les données n'ayant pas été classées précédemment, lorsque les abondances de taxons métagénomiques de la donnée métagénomique ne respectent aucune règle d'aucune sous-signature de l'ensemble de sous-signatures métagénomiques, ou les données ayant été classées dans deux classes antinomiques, par exemple dans une classe correspondant à la présence d'une pathologie et dans une classe correspondant à l'absence d'une pathologie, sont déléguées à une étape 18 à un procédé de classification supervisé de l'état de l'art. Les données n'ayant pas été classées ou ayant été classées dans deux classes antinomiques sont dites « rejetées ». Une donnée métagénomique peut en effet être rejetée si elle comprend une abondance de taxon métagénomique respectant une règle d'une sous-signature associée à une présence de la pathologie et si elle comprend une abondance d'un autre taxon métagénomique respectant une autre règle d'une autre sous-signature associée à une absence de pathologie. Dans un tel cas, a donnée métagénomique D est rejetée car le choix d'une classe parmi la présence ou l'absence de la pathologie est alors difficile à réaliser. Ces données rejetées sont déléguées à un procédé de classification de l'état de l'art tel qu'XGBoost, « Random Forest », « SVM » (pour « Support Vector Machine », ou « Machine à Vecteurs de Support » en français), ou tout autre procédé de classification supervisé de l'état de l'art. Cela permet de classifier les données rejetées par le procédé de classification basé sur l'ensemble S de sous-signatures propres à la pathologie, et d'ainsi améliorer la couverture du procédé de classification 1.

En phase de test, le procédé de classification 1 selon l'invention, hors délégation de classeur, permet de classer, dans l'exemple précédent des règles RP1 à RP5 et RN1 et RN2 apprises sur 232 patients, dont 118 cirrhotique et 114 sains :
- 97% des individus cirrhotiques par l'une des règles RP1 à RP5;
- 100% des non cirrhotiques sont classés par l'une des règles RN1 ou RN2 ;

Sur 47 patients de test, le procédé rejette 6 patients, soit 13% des patients, dont 4, soit 67%, sont ensuite correctement classés par le procédé de classification de l'état de l'art à l'étape 18.

Ainsi, la présente invention dans l'exemple de classification pour la pathologie « Cirrhose » a un « F1-score » de 94% et une « accuracy » de 94%, tout en donnant une explication (une ou des sous-signatures) pour 87% des individus classés, les 13% restants pouvant être classés par un procédé de classification de l'état de l'art (par exemple de « gradient boosting ») et 67% d'entre eux étant alors correctement prédits. Le F1-score est une moyenne pondérée entre la précision et le rappel, soit 2 * (précision * rappel) / (précision + rappel), le rappel étant le rapport entre le nombre d'individus d'une classe correctement prédits et le nombre total d'individus de cette classe, soit TP / (TP + FN), avec TP pour « True Positive » et FN pour « Faux Négatifs ».

## Revendications

1. Procédé (1) mis en œuvre par ordinateur de classification de patients, le procédé (1) étant **caractérisé en ce qu'**il comprend les étapes de :
- Réception (14) d'au moins une donnée métagénomique (D), la donnée métagénomique (D) étant le résultat d'une analyse métagénomique d'un échantillon d'un microbiome d'un patient et comprenant au moins une abondance de taxon métagénomique,
- Comparaison (16) de chaque abondance de taxon métagénomique de la donnée métagénomique (D) avec chaque sous-signature métagénomique d'au moins un ensemble (S) de sous-signatures métagénomiques propres à une pathologie, chaque sous-signature comprenant au moins une règle relative à une abondance de taxon métagénomique et étant associée à la présence ou à l'absence de la pathologie, l'ensemble (S) de sous-signatures métagénomiques ayant été généré par un procédé de découverte de sous-groupes,
- Si la donnée métagénomique (D) comprend au moins une abondance de taxon métagénomique respectant la règle d'au moins une sous-signature de l'ensemble (S) de sous-signatures métagénomiques, classification (17) du patient duquel la donnée métagénomique (D) est issue dans une classe représentative de la présence de la pathologie ou dans une classe représentative de l'absence de la pathologie en fonction de l'association de la sous-signature à la présence ou à l'absence de la pathologie.

2. Procédé (1) selon la revendication précédente **caractérisé en ce que**, si l'abondance de taxon métagénomique de la donnée métagénomique (D) ne respecte aucune règle d'aucune sous-signature de l'ensemble (S) de sous-signatures métagénomiques ou, si l'abondance de taxon métagénomique de la donnée métagénomique (D) respecte au moins une première et une deuxième règles respectivement d'une première et une deuxième sous-signatures de l'ensemble (S) de sous-signatures métagénomiques, la première sous-signature étant associée à la présence de la pathologie et la deuxième sous-signature étant associée à l'absence de la pathologie, le patient duquel la donnée métagénomique (D) est issue est classifié (18) par un procédé de classification supervisé (C).

3. Procédé (1) selon l'une quelconque des revendications précédentes **caractérisé en ce que** l'échantillon provient du microbiote intestinal du patient.

4. Procédé (1) selon l'une quelconque des revendications précédentes **caractérisé en ce que** la pathologie est une cirrhose, un cancer du côlon, un diabète, ou une maladie de Crohn.

5. Procédé (1) selon l'une quelconque des revendications précédentes **caractérisé en ce qu'**il comprend, après l'étape de réception de la donnée métagénomique (D), une étape de pré-traitement (15) de la donnée métagénomique (D) comprenant une transformation log-ratio centrée de la donnée métagénomique (D).

6. Procédé (1) selon l'une quelconque des revendications précédentes **caractérisé en ce qu'**il comprend préalablement les étapes suivantes:
- Une étape de réception (11) d'au moins une donnée métagénomique d'entraînement (Dde) de contrôle et d'au moins une donnée métagénomique d'entraînement (Dde) de cas, la donnée métagénomique d'entraînement (Dde) de contrôle étant le résultat d'une analyse métagénomique d'un échantillon d'un microbiote d'un patient cas-contrôle et la donnée métagénomique d'entraînement (Dde) de cas étant le résultat d'une analyse métagénomique d'un échantillon d'un microbiote d'un patient cas-témoin,
- Une étape de préparation (12) des données d'entraînement (Dde) reçues,
- Une étape de génération (13) de l'ensemble (S) de sous-signatures propres à la pathologie par le procédé de découverte de sous-groupes à partir des données métagénomiques d'entraînement (Dde) préparées.

7. Procédé (1) selon la revendication précédente **caractérisé en ce qu'**il comprend en outre au moins :
- Une étape d'optimisation (132) de l'ensemble de sous-signatures propres à la pathologie à partir des données métagénomiques d'entraînement (Dde) de contrôle pour obtenir au moins un jeu de sous-signatures (S1) propres à la pathologie optimisé pour les patients cas-contrôle,
- Une étape d'optimisation (133) de l'ensemble de sous-signatures propres à la pathologie à partir des données métagénomiques d'entraînement (Dde) de cas pour obtenir au moins un jeu de sous-signatures (S2) propres à la pathologie optimisé pour les patients cas-témoin,
- Une étape d'assemblage (134) du jeu de sous-signatures (S1) propres à la pathologie optimisée pour les patients cas-contrôle et du jeu de sous-signatures (S2) propres à la pathologie optimisée pour les patients cas-témoin pour obtenir l'ensemble de sous-signatures (S) propres à la pathologie.

8. Procédé (1) selon la revendication précédente **caractérisé en ce que** chaque étape d'optimisation comprend une pluralité de sous-étapes de calcul de métrique (M1, M2, M3), chaque sous-étape de calcul de métrique (M1, M2, M3) comprenant :
- le calcul d'une métrique pour chaque sous-signature propre à la pathologie de l'ensemble (S) de sous-signatures propres à la pathologie et
- la comparaison du résultat du calcul de ladite métrique avec une règle basée sur un seuil prédéfini,
chaque sous-étape (M1, M2, M3) étant associée à un niveau de contrainte, les sous-étapes (M1, M2, M3) se succédant du plus bas niveau de contrainte au plus haut niveau de contrainte, le jeu de sous-signatures (S1, S2) propres à la pathologie issu de l'étape d'optimisation (132, 133) comprenant au moins les sous-signatures propres à la pathologie respectant la règle basée sur un seuil prédéfini pour chaque sous-étape (M1, M2, M3) de la pluralité de sous-étapes (M1, M2, M3).

9. Procédé (1) selon la revendication précédente **caractérisé en ce que** chaque étape d'optimisation (132, 133) comprend en outre:
- Une sous-étape de sélection d'une première sous-signature parmi les sous-signatures comprises dans l'ensemble (S) de sous-signatures propres à la pathologie, la première sous-signature sélectionnée étant la sous-signature maximisant le résultat du calcul d'une métrique prédéfinie parmi les métriques calculées aux sous-étapes de calcul de métriques des étapes d'optimisation (132, 133),
- Pour chaque autre sous-signature de l'ensemble (S) de sous-signatures propres à la pathologie différente de la première sous-signature sélectionnée :
∘Une sous-étape de calcul de la métrique prédéfinie pour un groupe de sous-signatures comprenant la première sous-signature et l'autre sous-signature,
∘ Si la différence entre le résultat du calcul de la métrique pour le groupe de sous-signatures et le résultat du calcul de la métrique pour la première sous-signature respecte une règle basée sur un seuil de différence prédéfini, l'autre sous-signature est ajoutée au jeu de sous-signatures propres à la pathologie optimisé.

10. Produit-programme d'ordinateur comprenant des instructions qui, lorsque le programme est exécuté par un ordinateur, conduisent celui-ci à mettre en œuvre les étapes du procédé (1) de classification de patients selon l'une quelconque des revendications 1 à 9.

11. Support d'enregistrement lisible par ordinateur comprenant des instructions qui, lorsqu'elles sont exécutées par un ordinateur, conduisent celui-ci à mettre en œuvre les étapes du procédé (1) de classification de patients selon l'une quelconque des revendications 1 à 9.
